# EUROPEAN PATENT APPLICATION

(11) **EP 4 786 979 A1**
(43) Date of publication of application: **05.08.2026**
(21) Application number: 24872528.5
(22) Date of filing: 27.09.2024
(51) Int. Cl.: G01N 33/53, G01N 33/531, G01N 33/532, G01N 33/543

(54) **METHOD FOR MEASURING ANTIGEN CONTAINED IN TEST SAMPLE, AND KIT FOR MEASURING HUMAN CHORIONIC GONADOTROPIN OR ESTRADIOL**

(30) Priority: 29.09.2023 JP 2023170516
(71) Applicant: FUJIFILM Corporation, Tokyo 106-8620 (JP)
(72) Inventor: OUCHI Ryo, Tokyo 106-8620 (JP); KATO Aiko, Tokyo 106-8620 (JP); YOSHIMITSU Yuji, Tokyo 106-8620 (JP); OGURA Takahiro, Tokyo 106-8620 (JP)
(74) Representative: Dehns Germany Partnerschaft mbB
(86) International application number: PCT/JP2024/034708
(87) International publication number: WO 2025/070747

(57) **Abstract**

An object of the present invention is to provide a measurement method and a measurement kit, in which in measuring hCG or E2, the upper-limit concentration of the measurement is capable of being extended while making it possible to perform the luminescence measurement at the lower-limit concentration of the measurement. According to the present invention, there is provided a method for measuring an antigen contained in a test sample, the antigen being human chorionic gonadotropin or estradiol, the method including reacting, in the presence of one or more compounds represented by Formula (1), (1) an antigen having peroxidase bound to the antigen through a binding substance that recognizes the antigen, or an antigen having peroxidase bound to the antigen through a binding substance that recognizes a complex of the antigen and a binding substance that recognizes the antigen, (2) a chemiluminescent substance, and (3) an oxidizing agent.

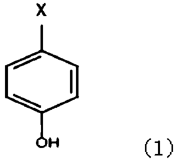

In the formula, X is a phenyl group, a pyridyl group, an oxazolyl group, a thiophenyl group, or a pyrrolyl group, which may have a substituent.

## Description

### Technical Field

The present invention relates to a method for measuring human chorionic gonadotropin or estradiol by using chemiluminescence, and a kit for measuring human chorionic gonadotropin or estradiol by using chemiluminescence.

### Background Art

In a chemiluminescence assay system using luminol or the like, it is known that thiazole phenol (TP) or 4-(4-hydroxyphenyl)benzoic acid (BiPCA) is used as an enhancer for enhancing chemiluminescence intensity.

Patent Document 1 describes BiPCA as an enhancer, but hCG and E2 are not measurement targets. US2015/0346202A describes that the effect of BiPCA is to maintain chemiluminescence and to extend a measurement time. In addition, Patent Document 2 describes BiPCA as an enhancer. However, the measurement of an antigen using BiPCA has not been conducted.

### Prior Art Documents

### Patent Documents

Patent Document 1: US2015/0346202A
Patent Document 2: JP-H7-311197A

### Summary of Invention

### Object to be solved by the invention

As a result of studies by the present inventors, in a case where human chorionic gonadotropin (hCG) and estradiol (E2) are measured in the presence of TP used as an enhancer in the related art, it is found that, as an issue specific to these antigens, the chemiluminescence intensity is enhanced, but the measurement cannot be performed over the entire range of the required antigen concentration. That is, in the measurement of hCG and E2 using TP as the enhancer, it is found that since the luminescence intensity at the lower-limit concentration of the measurement is enhanced, but the enhancement at the upper-limit concentration of the measurement is weak, it is not possible to extend the upper-limit concentration of the measurement while making it possible to perform the luminescence measurement at the lower-limit concentration of the measurement (to increase the luminescence intensity with an increase in the measurement concentration from the lower-limit of the measurement to the vicinity of the upper-limit of the measurement). Patent Documents 1 and 2 do not describe an object, in measuring hCG or E2, of extending the upper-limit concentration of the measurement while making it possible to perform the luminescence measurement at the lower-limit concentration of the measurement.

An object of the present invention is to provide a method for measuring hCG or E2, in which in measuring hCG or E2, the upper-limit concentration of the measurement is capable of being extended while making it possible to perform the luminescence measurement at the lower-limit concentration of the measurement. Another object of the present invention is to provide a kit for measuring hCG or E2, in which the upper-limit concentration of the measurement is capable of being extended while making it possible to perform the luminescence measurement at lower-limit concentration of the measurement.

### Means for solving the object

To solve the above-described issue, the present inventors have made intensive studies and screened various compounds, and as a result, found that the above-described issue can be solved by using BiPCA or the like as an enhancer in the measurement of hCG or E2. The present invention has been completed based on the above findings. That is, according to the present invention, the following invention is provided.
<1> A method for measuring an antigen contained in a test sample, the antigen being human chorionic gonadotropin or estradiol, the method comprising:
   reacting, in a presence of one or more compounds represented by Formula (1),
      (1) an antigen having peroxidase bound to the antigen through a binding substance that recognizes the antigen, or
         an antigen having peroxidase bound to the antigen through a binding substance that recognizes a complex of the antigen and a binding substance that recognizes the antigen,
      (2) a chemiluminescent substance, and
      (3) an oxidizing agent, 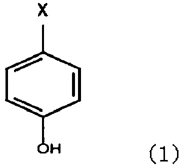
   in the formula, X is
      a phenyl group that is unsubstituted or has a substituent,
      a pyridyl group that is unsubstituted or has a substituent,
      an oxazolyl group that is unsubstituted or has a substituent,
      a thiophenyl group that is unsubstituted or has a substituent, or
      a pyrrolyl group that is unsubstituted or has a substituent, and
   the substituent is a carboxyl group, an amide group, a halogen atom, an alkoxy group having 1 to 5 carbon atoms, or an alkyl group having 1 to 5 carbon atoms.
<2> The method according to <1>, in which X is
   a phenyl group having a carboxyl group, an amide group, a halogen atom, or an alkoxy group having 1 to 5 carbon atoms,
   a pyridyl group that is unsubstituted or has an alkyl group having 1 to 5 carbon atoms or an alkoxy group having 1 to 5 carbon atoms,
   an oxazolyl group that is unsubstituted or has an alkyl group having 1 to 5 carbon atoms or an alkoxy group having 1 to 5 carbon atoms,
   a thiophenyl group that is unsubstituted or has a carboxyl group, or
   a pyrrolyl group that is unsubstituted or has an alkyl group having 1 to 5 carbon atoms or an alkoxy group having 1 to 5 carbon atoms.
<3> The method according to <1> or <2>, in which X is a phenyl group having a carboxyl group, an amide group, a halogen atom, or an alkoxy group having 1 to 5 carbon atoms.
<4> The method according to any one of <1> to <3>, in which the antigen having peroxidase bound to the antigen through a binding substance that recognizes the antigen is an antigen obtained by reacting the antigen with a first binding substance that recognizes the antigen to form a complex, and subsequently reacting the complex with a second binding substance that recognizes the antigen, the second binding substance having peroxidase bound to the antigen.
<5> The method according to any one of <1> to <3>, in which the antigen having peroxidase bound to the antigen through a binding substance that recognizes a complex of the antigen and a binding substance that recognizes the antigen is an antigen obtained by reacting the antigen with a first binding substance that recognizes the antigen to form a complex, and subsequently reacting the complex with "a third binding substance that recognizes the complex of the antigen and the first binding substance that recognizes the antigen", the third binding substance having peroxidase bound to the antigen.
<6> The method according to any one of <1> to <5>, in which a concentration of the compound represented by Formula (1) is 10 µmol/L or more and 500 µmol/L or less.
<7> The method according to any one of <1> to <6>, in which the chemiluminescent substance is luminol, a luminol derivative, 8-amino-5-chloro-7-phenylpyrido[3,4-d]pyridazine-1,4(2H,3H)-dione sodium salt, or salts thereof.
<8> The method according to <7>, in which a concentration of the chemiluminescent substance is 0.1 mmol/L or more and 10 mmol/L or less.
<9> The method according to any one of <1> to <8>, in which the oxidizing agent is hydrogen peroxide.
<10> A kit for measuring human chorionic gonadotropin or estradiol, the kit comprising:
   peroxidase to which a binding substance that recognizes an antigen is bound;
   a chemiluminescent substance;
   an oxidizing agent; and
   one or more compounds represented by Formula (1), 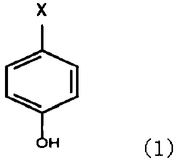
   in the formula, X is
      a phenyl group that is unsubstituted or has a substituent,
      a pyridyl group that is unsubstituted or has a substituent,
      an oxazolyl group that is unsubstituted or has a substituent,
      a thiophenyl group that is unsubstituted or has a substituent, or
      a pyrrolyl group that is unsubstituted or has a substituent, and
   the substituent is a carboxyl group, an amide group, a halogen atom, an alkoxy group having 1 to 5 carbon atoms, or an alkyl group having 1 to 5 carbon atoms.
<11> The kit according to <10>, further comprising:
   another binding substance that recognizes a measurement antigen, the other binding substance being different from the binding substance bound to the peroxidase; or
   a solid-phase carrier on which the other binding substance is immobilized.
<12> A kit for measuring human chorionic gonadotropin or estradiol, the kit comprising:
   peroxidase to which a binding substance that recognizes a complex of an antigen and a binding substance that recognizes the antigen is bound;
   a chemiluminescent substance;
   an oxidizing agent; and
   one or more compounds represented by Formula (1), 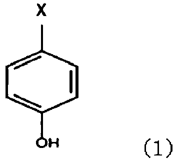
   in the formula, X is
      a phenyl group that is unsubstituted or has a substituent,
      a pyridyl group that is unsubstituted or has a substituent,
      an oxazolyl group that is unsubstituted or has a substituent,
      a thiophenyl group that is unsubstituted or has a substituent, or
      a pyrrolyl group that is unsubstituted or has a substituent, and
   the substituent is a carboxyl group, an amide group, a halogen atom, an alkoxy group having 1 to 5 carbon atoms, or an alkyl group having 1 to 5 carbon atoms.
<13> The kit according to <12>, further comprising:
   another binding substance that recognizes a measurement antigen, the other binding substance being different from the binding substance bound to the peroxidase; or
   a solid-phase carrier on which the other binding substance is immobilized.

### Effect of the invention

According to the measurement method and the measurement kit of the present invention, in measuring hCG or E2, the upper-limit concentration of the measurement is capable of being extended while making it possible to perform the luminescence measurement at the lower-limit concentration of the measurement.

### BRIEF DESCRIPTION OF THE DRAWINGS

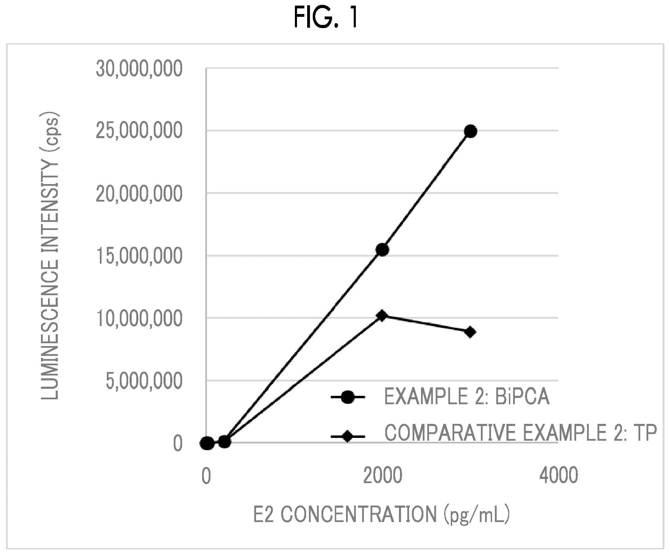

[FIG. 1] shows a result of measuring a luminescence intensity at each E2 concentration in Example 2 and Comparative Example 2.

### Embodiments for carrying out the invention

Hereinafter, the present invention will be described in detail. In the present specification, mM indicates mmol/L.

### <Method for measuring antigen>

A first aspect of the present invention relates to a method for measuring an antigen contained in a test sample, the antigen being human chorionic gonadotropin or estradiol, the method including:
reacting, in the presence of one or more compounds represented by Formula (1),
   (1) an antigen having peroxidase bound to the antigen through a binding substance that recognizes the antigen, or
      an antigen having peroxidase bound to the antigen through a binding substance that recognizes a complex of the antigen and a binding substance that recognizes the antigen,
   (2) a chemiluminescent substance, and
   (3) an oxidizing agent, 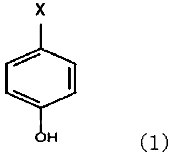
in the formula, X is
   a phenyl group that is unsubstituted or has a substituent,
   a pyridyl group that is unsubstituted or has a substituent,
   an oxazolyl group that is unsubstituted or has a substituent,
   a thiophenyl group that is unsubstituted or has a substituent, or
   a pyrrolyl group that is unsubstituted or has a substituent, and
the substituent is a carboxyl group, an amide group, a halogen atom, an alkoxy group having 1 to 5 carbon atoms, or an alkyl group having 1 to 5 carbon atoms.

Examples of the test sample include biological samples such as bodily fluids such as serum, blood, blood plasma, and urine, lymphatic fluid, blood cells, and various cells, but the test sample is not particularly limited.

In the present invention, the antigen contained in the test sample is human chorionic gonadotropin or estradiol.

Preferably, in Formula (1), X is
a phenyl group having a carboxyl group, an amide group, a halogen atom, or an alkoxy group having 1 to 5 carbon atoms,
a pyridyl group that is unsubstituted or has an alkyl group having 1 to 5 carbon atoms or an alkoxy group having 1 to 5 carbon atoms,
an oxazolyl group that is unsubstituted or has an alkyl group having 1 to 5 carbon atoms or an alkoxy group having 1 to 5 carbon atoms,
a thiophenyl group that is unsubstituted or has a carboxyl group, or
a pyrrolyl group that is unsubstituted or has an alkyl group having 1 to 5 carbon atoms or an alkoxy group having 1 to 5 carbon atoms.

More preferably, in Formula (1), X is a phenyl group having a carboxyl group, an amide group, a halogen atom, or an alkoxy group having 1 to 5 carbon atoms.

Examples of the halogen atom include fluorine, chlorine, bromine, and iodine, and fluorine or chlorine is preferable.

Examples of the alkoxy group having 1 to 5 carbon atoms include a methoxy group, an ethoxy group, a propoxy group, a butoxy group, and a pentyloxy group, which may be linear or branched. The alkoxy group having 1 to 5 carbon atoms is preferably a methoxy group.

Examples of the alkyl group having 1 to 5 carbon atoms include a methyl group, an ethyl group, a propyl group, a butyl group, and a pentyl group, which may be linear or branched. The alkyl group having 1 to 5 carbon atoms is preferably a methyl group.

Specific examples of the group represented by X are shown below. Among the following, the group represented by X is particularly preferably a group surrounded by a square frame. * indicates a bonding position to a benzene ring.
(1) Phenyl group having a substituent 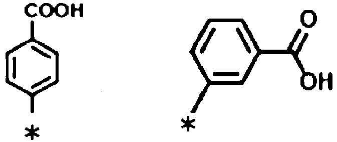 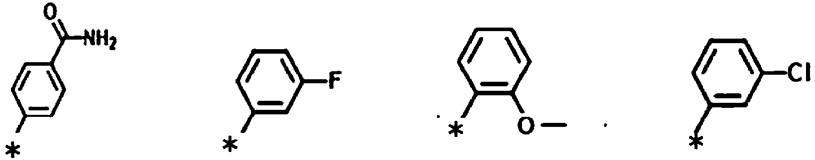
(2) Pyridyl group unsubstituted or having a substituent 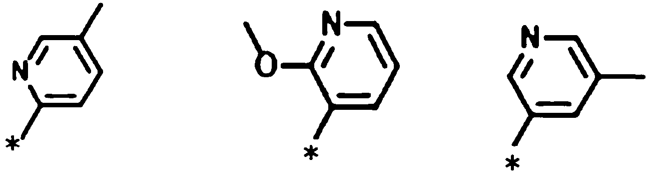 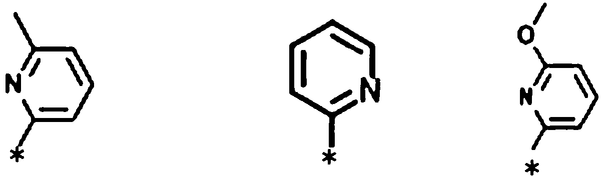
(3) Oxazolyl group unsubstituted or having a substituent 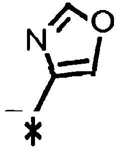
(4) Thiophenyl group unsubstituted or having a substituent 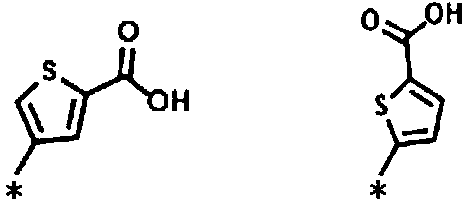
(5) Pyrrolyl group unsubstituted or having a substituent

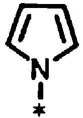

Specific examples of the compound represented by Formula (1) are shown below.

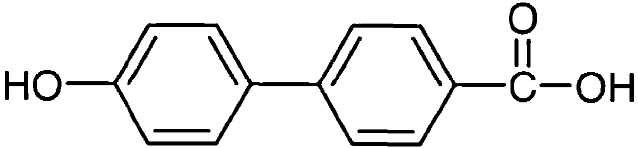

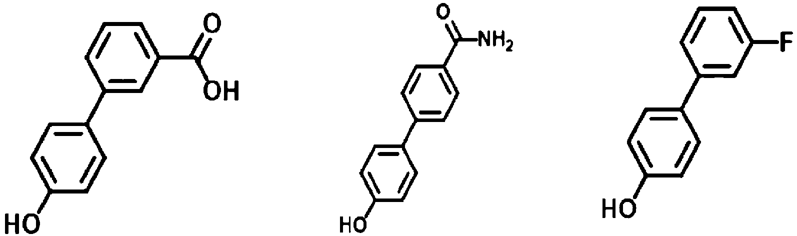

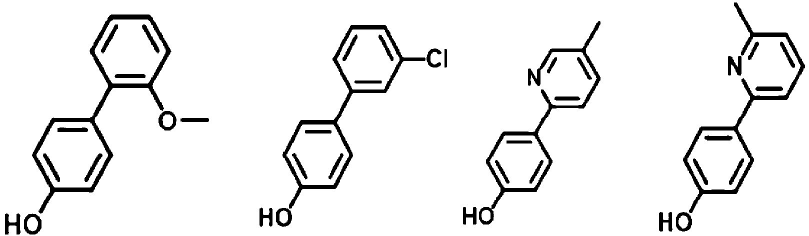

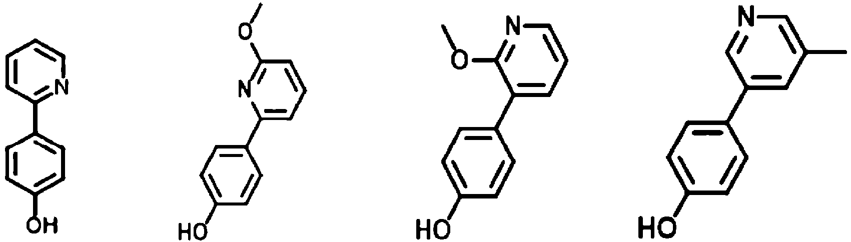

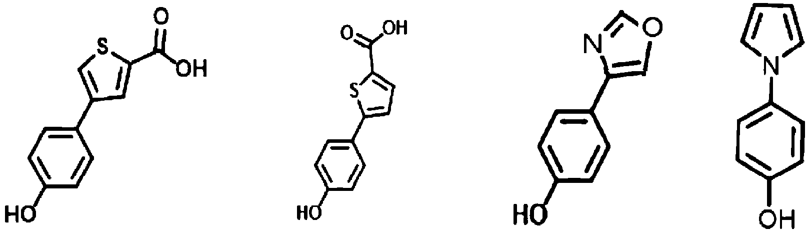

A concentration of the compound represented by Formula (1) is preferably 10 µmol/L or more and 500 µmol/L or less, more preferably 50 µmol/L or more and 500 µmol/L or less, still more preferably 75 µmol/L or more and 400 µmol/L or less, and particularly preferably 100 µmol/L or more and 300 µmol/L or less.

The concentration referred to here is a concentration in a reaction solution in a case where the antigen having peroxidase bound to the antigen through a binding substance that recognizes the antigen, the chemiluminescent substance, and the oxidizing agent are reacted in the presence of one or more compounds represented by Formula (1).

In the present invention, the antigen having peroxidase bound to the antigen through a binding substance that recognizes the antigen, the chemiluminescent substance, and the oxidizing agent are reacted in the presence of one or more compounds represented by Formula (1).

The chemiluminescent substrate is preferably a nitrogen-containing heterocyclic compound having an amino group or a salt thereof, and examples thereof include a 2,3-dihydro-1,4-phthalazinedione compound having an amino group, and 8-amino-5-chloro-7-phenylpyrido[3,4-d]pyridazine-1,4(2H,3H)-dione sodium salt (L-012) or a salt thereof.

The structure of L-012 is shown below.

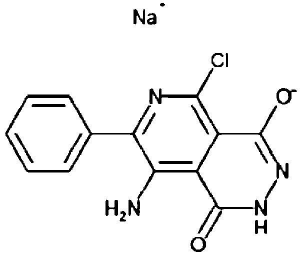

As the 2,3-dihydro-1,4-phthalazinedione compound, for example, known 2,3-dihydro-1,4-phthalazinedione compounds described in JP1990-291299A (JP-H2-291299A), JP1998-319015A (JP-H10-319015A), JP2000-279196A, mixtures thereof, and the like can be used. Among these, luminol, a luminol derivative (for example, isoluminol, N-aminohexyl-N-ethylisoluminol (AHEI), N-aminobutyl-N-ethylisoluminol (ABEI), and the like), and metal salts thereof (an alkali metal salt and the like) are preferable, and luminol and a metal salt thereof are more preferable, and a sodium salt of luminol is particularly preferable.

A concentration of the chemiluminescent substance is preferably 0.1 mmol/L or more and 10 mmol/L or less, more preferably 0.2 mmol/L or more and 7.5 mmol/L or less, still more preferably 0.25 mmol/L or more and 5.0 mmol/L or less, and particularly preferably 0.25 mmol/L or more and 2.5 mmol/L or less.

The concentration referred to here is a concentration in a reaction solution in a case where the antigen having peroxidase bound to the antigen through a binding substance that recognizes the antigen, the chemiluminescent substance, and the oxidizing agent are reacted in the presence of one or more compounds represented by Formula (1).

Examples of the oxidizing agent include aqueous solutions of known oxidizing agents described in JP1996-261943A (JP-H8-261943A) and JP2000-279196A and the like [inorganic peroxides (such as hydrogen peroxide, sodium perborate, and potassium perborate), organic peroxides (such as dialkyl peroxide and acyl peroxide), and peroxo acid compounds (such as peroxosulfuric acid and peroxophosphoric acid)]. Among these, from the viewpoint of storage stability and the like, hydrogen peroxide, sodium perborate, and potassium perborate are preferable, and hydrogen peroxide is more preferable.

A concentration of the oxidizing agent is appropriately set depending on the type thereof and a measurement method, a measurement condition, and the like to be applied, but from the viewpoint of a chemiluminescence enhancement effect and the like, it is preferably 0.5 mmol/L or more and 20 mmol/L or less, more preferably 1 mmol/L or more and 10 mmol/L or less, and still more preferably 1.4 mmol/L or more and 3.5 mmol/L or less.

The concentration referred to here is a concentration in a reaction solution in a case where the antigen having peroxidase bound to the antigen through a binding substance that recognizes the antigen, the chemiluminescent substance, and the oxidizing agent are reacted in the presence of one or more compounds represented by Formula (1).

In one example of the present invention, the antigen having peroxidase bound to the antigen through a binding substance that recognizes the antigen may be an antigen obtained by reacting the antigen with a first binding substance that recognizes the antigen to form a complex, and subsequently reacting the complex with a second binding substance that recognizes the antigen, the second binding substance having peroxidase bound to the antigen.

In one example of the present invention, the antigen having peroxidase bound to the antigen through a binding substance that recognizes a complex of the antigen and a first binding substance that recognizes the antigen may be an antigen obtained by reacting the antigen with a first binding substance that recognizes the antigen to form a complex, and subsequently reacting the complex with "a third binding substance that recognizes the complex of an antigen and a first binding substance that recognizes the antigen", the third binding substance having peroxidase bound to the antigen.

In the present invention, for example, the antigen in a sample can be measured using a solid-phase carrier on which a first binding substance that recognizes an antigen is immobilized on a surface of a solid-phase carrier such as magnetic silica particles.

Examples of the binding substance that recognizes an antigen (the first binding substance that recognizes an antigen or the second binding substance that recognizes an antigen) include substances that bind to human chorionic gonadotropin or estradiol, which is a measurement target substance, by mutual reactions such as an "antigen"-"antibody" reaction, a "glycan"-"protein" reaction, a "glycan"-"lectin" reaction, a "protein"-"peptide chain" reaction, a "protein"-"protein" reaction, and a "protein"-"nucleotide chain" reaction.

Examples of the binding substance that recognizes the above-described complex include substances that bind to the complex by mutual reactions such as an "antigen"-"antibody" reaction, a "glycan"-"protein" reaction, a "glycan"-"lectin" reaction, a "protein"-"peptide chain" reaction, a "protein"-"protein" reaction, and a "protein"-"nucleotide chain" reaction.

The measurement target substance measurement method according to the embodiment of the present invention may be performed according to a measurement method of a sandwich method described in a document [for example, Enzyme Immunoassay, 2nd Edition (edited by Eiji Ishikawa, Igaku Shoin) 1982] usually performed in the field.

In the sandwich method, for example, a first binding substance that recognizes an antigen is immobilized on a surface of a solid-phase carrier such as magnetic silica particles, a sample containing an antigen, the solid-phase carrier, and a second binding substance that recognizes an antigen and is labeled with peroxidase are mixed, and the immobilized first binding substance, the antigen in the sample, and the second binding substance are brought into contact with each other. As a result, a peroxidase-labeled complex, which is a complex of the immobilized first binding substance, the antigen in the sample, and the second binding substance labeled with peroxidase, is formed. Next, the solid-phase carrier carrying the peroxidase-labeled complex is separated into a B/F fraction, the amount of peroxidase in the peroxidase-labeled complex is measured, and the amount of the antigen in the sample is measured based on the amount of peroxidase in the peroxidase-labeled complex.

Alternatively, for example, a first binding substance that recognizes an antigen is immobilized on a surface of a solid-phase carrier such as magnetic silica particles, a sample containing an antigen, the solid-phase carrier, and "a third binding substance that recognizes a complex of an antigen and a first binding substance that recognizes the antigen", the third binding substance being labeled with peroxidase, are mixed, and the immobilized first binding substance, the antigen in the sample, and the third binding substance are brought into contact with each other. As a result, a peroxidase-labeled complex, which is a complex of the immobilized first binding substance, the antigen in the sample, and the third binding substance labeled with peroxidase, is formed. Next, the solid-phase carrier carrying the peroxidase-labeled complex is separated into a B/F fraction, the amount of peroxidase in the peroxidase-labeled complex is measured, and the amount of the antigen in the sample is measured based on the amount of peroxidase in the peroxidase-labeled complex.

As the solid-phase carrier, any support (particularly, an insoluble support) used in a normal immunological assay method can be used. Examples thereof include organic substances such as polystyrene, carboxylated polystyrene, polyacrylic acid, polymethacrylic acid, methyl polymethacrylate, polyacrylamide, polyglycidyl methacrylate, polypropylene, polyolefin, polyimide, polyurethane, polyester, polyvinyl chloride, polyethylene, polychlorocarbonate, a silicone resin, a silicone rubber, agarose, dextran, an ethylene-maleic anhydride copolymer, and the like; inorganic substances such as glass, silicon oxides, diatom, porous glass, obscured glass, alumina, silica gel, metal oxides, and the like; magnetic materials such as iron, cobalt, nickel, magnetite, chromite, and the like; and those prepared using an alloy of these magnetic materials as a material.

The immobilization can be performed on a solid phase (for example, any surface such as beads, magnetic beads, a membrane, and a plate). As the magnetic beads, various commercially available magnetic beads can be used. As the magnetic beads, for example, beads disclosed in WO2012/173002A may be used.

Specifically, for example, the antigen in the sample and the first binding substance that recognizes the antigen and is immobilized on a surface of a solid-phase carrier such as magnetic silica particles are brought into contact with each other to form a complex of the first binding substance that recognizes the antigen and is immobilized on the surface of the solid-phase carrier and the antigen in the sample. Next, the complex is brought into contact with a complex of the second binding substance labeled with peroxidase to form a complex (peroxidase-labeled complex) of the first binding substance that recognizes the antigen and is immobilized on the solid-phase carrier, the antigen in the sample, and the second binding substance labeled with peroxidase. The peroxidase-labeled complex can be separated into a B/F fraction, the amount of peroxidase in the peroxidase-labeled complex can be measured, and the amount of the antigen in the sample can be measured based on the amount of peroxidase in the peroxidase-labeled complex.

In the above-described method, after reacting the antigen in the sample with the immobilized first binding substance that recognizes the antigen, the second binding substance labeled with peroxidase is reacted thereto. However, the immobilized first binding substance that recognizes the antigen may be reacted after reacting the antigen in the sample with the second binding substance labeled with peroxidase, or all of these three may be reacted at the same time.

The B/F separation (bond/free separation) in the above-described sandwich method means separation of a substance carried on the solid-phase carrier and other substances. That is, the B/F separation in the present invention is
(1) separation of "a complex of the first binding substance recognizes an antigen and is immobilized on a surface of a solid-phase carrier and the antigen in a sample" and an antigen or other substances in a reaction system, which does not participate in the formation of the complex, and
(2) separation of a peroxidase-labeled complex and a second binding substance labeled with peroxidase or other substances in a reaction system, which does not participate in the formation of the peroxidase-labeled complex.

The sandwich method can also be performed by a microfluidic chip (micro-total analysis system; µTAS). The microfluidic chip includes a flow path, and the flow path has a sample introduction port. For example, at the sample introduction port, the first binding substance that recognizes an antigen and is bound to a polycation or a polyanion (for example, DNA) is brought into contact with the antigen in the sample to form a complex of the first binding substance that recognizes the antigen and the antigen. Next, the complex is moved by applying an electric field, and in a case where the second binding substance labeled with peroxidase and the complex, which are present in the middle of the flow path, come into contact with each other, a complex (peroxidase-labeled complex) of the first binding substance, the antigen, and the second binding substance labeled with peroxidase is formed. Furthermore, the peroxidase-labeled complex is moved in the flow path by the electric field, the peroxidase-labeled complex is separated into a B/F fraction, and the peroxidase-labeled complex reaches a detection unit on the flow path. In the detection unit, the amount of peroxidase in the peroxidase-labeled complex is measured, and the amount of the antigen in the sample may be measured based on the amount of peroxidase in the peroxidase-labeled complex.

In the measurement method according to the embodiment of the present invention, the antigen in the sample may be brought into contact with the first binding substance that recognizes the antigen and is immobilized on the surface of the solid-phase carrier by a usually performed treatment such as stirring and mixing. A reaction time may be appropriately set depending on the antigen and the first binding substance, but is usually 1 minute to 24 hours, preferably 1 minute to 1 hour, more preferably 1 to 10 minutes, and particularly preferably 1 to 5 minutes.

The B/F separation in the measurement method according to the embodiment of the present invention is performed, for example, by using the magnetism of the solid-phase carrier, collecting the solid-phase carrier from the outside of a reaction tank or the like with a magnet or the like, discharging the reaction solution, adding a washing solution, removing the magnet, mixing and dispersing the solid-phase carrier, and washing the solid-phase carrier. The above-described operation may be repeated 1 to 3 times. The washing solution is not particularly limited as long as it is usually used in the field.

As the peroxidase, horseradish peroxidase (HRP), microperoxidase, or the like can be used.

To bond the peroxidase to the second binding substance or the third binding substance, a method usually used in the field, for example, a known labeling method generally performed in a known EIA [for example, Ikagaku Jikken Koza, Vol. 8, supervised by Yuichi Yamamura, 1st ed., Nakayama Shoten, 1971; Zusetsu Keiko Kotai (or: Zusetsu Keiko Kotaiho), Akira Kawao, 1st ed., Soft Science-sha, 1983; Koso Meneki Sokuteiho, edited by Eiji Ishikawa, Tadashi Kawai, and Kiyoshi Miyai, 2nd ed., Igaku-Shoin, 1982; and the like] may be used.

As the amount of the peroxidase used, it is preferable that the second binding substance or the third binding substance and the peroxidase are used, for example, in a molar ratio of usually 1:0.5 to 20, preferably 1:1 to 15, and more preferably 1:1 to 10. In addition, the binding substance that recognizes the antigen labeled with peroxidase may be used by being contained in a buffer solution usually used in the field, such as a Tris buffer solution, a phosphate buffer solution, a veronal buffer solution, a borate buffer solution, a Good's buffer solution (for example, a MES (2-morpholinoethanesulfonic acid) buffer solution or the like), or the like.

A concentration of the peroxidase to which the second binding substance or the third binding substance is bound is not particularly limited, but is preferably 0.1 nmol/L or more and 5,000 nmol/L or less, more preferably 1 nmol/L or more and 500 nmol/L or less, still more preferably 3 nmol/L or more and 300 nmol/L or less, and particularly preferably 5 nmol/L or more and 150 nmol/L or less.

In a case where an antibody is used as the binding substance, the pH of the buffer solution may be in a range that does not suppress the antigen-antibody reaction, and is usually 5 to 9. In addition, the buffer solution may contain, for example, a stabilizer such as albumin, globulin, water-soluble gelatin, and polyethylene glycol, a surfactant, and sugars, as long as the antigen-antibody reaction is not inhibited. It is noted that in the present specification, the pH means a numerical value measured in accordance with JIS K0400-12-10:2000 (measurement temperature of 25°C).

### <Measurement kit of human chorionic gonadotropin or estradiol>

A second aspect of the present invention relates to a kit for measuring human chorionic gonadotropin or estradiol, the kit including peroxidase to which a binding substance that recognizes an antigen is bound, a chemiluminescent substance, an oxidizing agent, and one or more compounds represented by Formula (1).

A third aspect of the present invention relates to a kit for measuring human chorionic gonadotropin or estradiol, the kit including peroxidase to which a binding substance that recognizes a complex of the antigen and a binding substance that recognizes the antigen is bound, a chemiluminescent substance, an oxidizing agent, and one or more compounds represented by Formula (1).

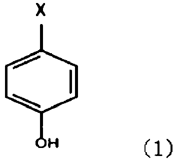

In the formula, X is
a phenyl group that is unsubstituted or has a substituent,
a pyridyl group that is unsubstituted or has a substituent,
an oxazolyl group that is unsubstituted or has a substituent,
a thiophenyl group that is unsubstituted or has a substituent, or
a pyrrolyl group that is unsubstituted or has a substituent, and
the substituent is a carboxyl group, an amide group, a halogen atom, an alkoxy group having 1 to 5 carbon atoms, or an alkyl group having 1 to 5 carbon atoms.

Preferred aspects and specific examples of each of the peroxidase to which a binding substance that recognizes an antigen is bound, the chemiluminescent substance, the oxidizing agent, and the one or more compounds represented by Formula (1) are as described above in the present specification. In addition, the peroxidase to which a binding substance that recognizes an antigen is bound, the peroxidase to which a binding substance that recognizes a complex of an antigen and a binding substance that recognizes the antigen is bound, the chemiluminescent substance, the oxidizing agent, and the one or more compounds represented by Formula (1) in the present reagent kit may be contained in the reagent such that the concentration thereof is as described above in the present specification at the time of the reaction.

In the kit of the present invention, a concentration (concentration as a reagent) of the chemiluminescent substance is not particularly limited, but is preferably 0.2 mmol/L or more and 20 mmol/L or less, more preferably 0.4 mmol/L or more and 15.0 mmol/L or less, still more preferably 0.5 mmol/L or more and 10.0 mmol/L or less, and particularly preferably 0.5 mmol/L or more and 5.0 mmol/L or less.

In the kit of the present invention, a concentration (concentration as a reagent) of the oxidizing agent is not particularly limited, but is preferably 1.0 mmol/L or more and 40 mmol/L or less, more preferably 2 mmol/L or more and 20 mmol/L or less, and still more preferably 2.8 mmol/L or more and 7.0 mmol/L or less.

Concentrations of the one or more of compounds represented by Formula (1) are preferably 20 µmol/L or more and 1,000 µmol/L or less, more preferably 100 µmol/L or more and 1,000 µmol/L or less, still more preferably 150 µmol/L or more and 800 µmol/L or less, and particularly preferably 200 µmol/L or more and 600 µmol/L or less.

In the kit according to the embodiment of the present invention, a concentration (concentration as a reagent) of the peroxidase to which the second binding substance or the third binding substance is bound is not particularly limited, but is preferably 0.1 nmol/L or more and 5,000 nmol/L or less, more preferably 1 nmol/L or more and 500 nmol/L or less, still more preferably 3 nmol/L or more and 300 nmol/L or less, and particularly preferably 5 nmol/L or more and 150 nmol/L or less.

The measurement kit according to the embodiment of the present invention may further include another binding substance that recognizes a measurement antigen, the other binding substance being different from the binding substance bound to the peroxidase, or a solid-phase carrier on which the other binding substance is immobilized.

The present invention will be more specifically described using the following examples; however, it is not limited by the examples.

### Examples

### <Synthesis Examples> Synthesis of various compounds

Purification by silica gel column chromatography was performed using an automatic purification device ISOLERA (manufactured by Biotage AB).

As a carrier in normal phase chromatography, CHROMATOREX Q-PACK (manufactured by Fuji Silysia Chemical Ltd.) was used.

As a carrier in reverse phase chromatography, Sfar C18 (manufactured by Biotage AB) was used.

Mass (MS) spectrum was measured using ACQUITY SQD LC/MS System (manufactured by Waters Corporation), ionization method, electrospray ionization (ESI) method.

The retention time (RT) was measured using a SQD (Waters Corporation), and was shown in minutes (min).
Column: BEH C18 1.7 µm, 2.1 × 30 mm, manufactured by Waters Corporation
Solvent: liquid A: 0.1% formic acid-water
Liquid B: 0.1% formic acid-acetonitrile
Gradient cycle: 0.00 min (liquid A/liquid B = 95/5), 2.00 min (liquid A/liquid B = 5/95), 3.00 min (liquid A/liquid B = 95/5)
Flow rate: 0.5 mL/min
Column temperature: 40°C
Detection wavelength: 254 nm

### Synthesis of 3'-fluoro-[1,1'-biphenyl]-4-ol

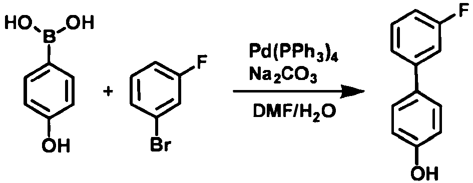

A solution of 1-bromo-3-fluorobenzene (93.0 mg) in N,N-dimethylformamide (1.0 mL), sodium carbonate (154 mg), water (100 µL), and tetrakis(triphenylphosphine)palladium (168 mg) was added to 4-hydroxyphenylboronic acid (100 mg), and the mixture was stirred at 120°C for 1 hour under a nitrogen atmosphere using Initiator^{™} (Biotage AB). The reaction solution was purified by normal phase column chromatography (ethyl acetate/hexane = 0/100 → 100/0) and reverse phase column chromatography (0.1% formic acid-acetonitrile/0.1% formic acid-water = 0/100 → 100/0) to obtain 3'-fluoro-[1,1'-biphenyl]-4-ol as a white solid (31.9 mg).
MS (ESI m/z): 187.3 (M-H)
RT (min): 1.37

The compounds shown in Table 1 were synthesized in the same manner as in the synthesis of 3'-fluoro-[1,1'-biphenyl]-4-ol.

**[Table 1]**

| Structure | Compound name | MS (ESI m/z) | RT/min |
|---|---|---|---|
| 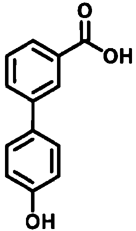 | 4'-Hydroxy-[1,1'-biphenyl]-3-carboxylic acid | 213.4 | 1.01 |
| 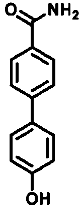 | 4'-Hydroxy-[1,1'-biphenyl]-4-carboxamide | 212.4 | 0.77 |
| 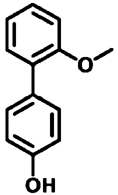 | 2'-Methoxy-[1,1'-biphenyl]-4-ol | 201.4 | 1.31 |
| 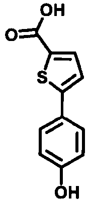 | 5-(4-hydroxyphenyl)thiophen-2-carboxylic acid | 221.3 | 0.95 |
| 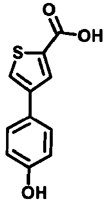 | 4-(4-hydroxyphenyl)thiophen-2-carboxylic acid | 221.3 | 0.95 |
| 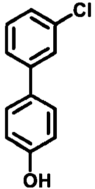 | 3'-chloro-[1,1'-biphenyl]-4-ol | 203.4 | 1.48 |
| 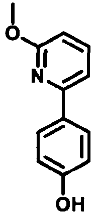 | 4-(6-methoxypyridin-2-yl)phenol | 202.4 | 0.48 |
| 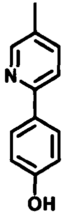 | 4-(5-methylpyridin-2-yl)phenol | 186.4 | 0.54 |
| 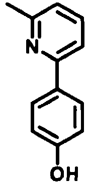 | 4-(6-methylpyridin-2-yl)phenol | 186.4 | 0.49 |
| 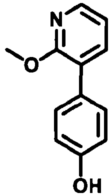 | 4-(2-methoxypyridin-3-yl)phenol | 202.4 | 1.11 |
| 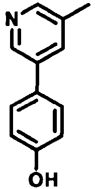 | 4-(5-methylpyridin-3-yl)phenol | 186.4 | 0.54 |

### <Example 1> Evaluation of BiPCA in measurement of hCG

### 1. Preparation of luminescent reagent (hCG)

A constitutional reagent required for the measurement was prepared using the following reagent raw materials.

MES (2-morpholinoethanesulfonic acid monohydrate) (manufactured by Dojindo Laboratories), sodium chloride (manufactured by FUJIFILM Wako Pure Chemical Corporation), BCN300S (manufactured by Nitta Gelatin Inc.), Block Ace (manufactured by KAC Co., Ltd.), BSA (bovine serum albumin) (manufactured by Sigma-Aldrich Japan G.K.), sodium orthovanadate (manufactured by Sigma-Aldrich Japan G.K.), sodium luminol salt (manufactured by FUJIFILM Wako Pure Chemical Corporation), phosphoric acid (manufactured by FUJIFILM Wako Pure Chemical Corporation), EDTA-2Na (manufactured by Dojindo Laboratories), hydrogen peroxide (manufactured by FUJIFILM Wako Pure Chemical Corporation), and dipotassium hydrogen phosphate (manufactured by FUJIFILM Wako Pure Chemical Corporation).

### (i) "First reagent" magnetic particle-immobilized antibody-containing reagent

MAGRAPID MGP-010T (manufactured by Sanyo Chemical Industries, Ltd.) was sequentially reacted with 3-aminopropyltriethoxysilane (manufactured by Tokyo Chemical Industry Co., Ltd.) and succinic anhydride (manufactured by FUJIFILM Wako Pure Chemical Corporation), and then magnetic particles were collected with a neodymium magnet and the supernatant was removed to obtain magnetic particles having a carboxyl group. Next, the carboxyl group was activated by commercially available N-hydroxysuccinimide (manufactured by FUJIFILM Wako Pure Chemical Corporation) and water-soluble carbodiimide (WSC) (manufactured by Dojindo Laboratories), and then Mab to hCG beta (manufactured by Meridian Japan Co., Ltd.), which is an anti-hCG antibody, was reacted therewith at 26°C for 12 to 16 hours, and the magnetic particles were collected with a neodymium magnet and the supernatant was removed to prepare magnetic particles immobilized with an anti-hCG antibody, thereby preparing a first reagent consisting of the following composition.

"First reagent": 0.5 mg/mL of magnetic particles immobilized with an anti-hCG antibody, 50 mM MES (pH 5.5), 500 mM sodium chloride, 3.0% BCN300S
(ii) "Second reagent" reaction buffer solution: 50 mM MOPS (3-morpholinopropanesulfonic acid) (manufactured by Dojindo Laboratories) (pH 7.5), 500 mM sodium chloride, 0.4% Block Ace
(iii) "Third reagent" label antibody-containing reagent

MAB <HCG> M-INN22 IgG (manufactured by Roche Diagnostics K.K.), which is an anti-hCG antibody, was digested with pepsin, and F(ab')2 was separated by a column (diameter: 1.5 cm × length: about 95 cm) filled with Sephacryl S-200HR (manufactured by Cytiva). The obtained F(ab')2 was reduced with cysteamine hydrochloride (manufactured by Sigma-Aldrich Japan G.K.), and Fab' was separated by a column (diameter: 1.5 cm × length: about 40 cm) filled with G-25 Superfine (manufactured by Cytiva). On the other hand, using a maleimide reagent Sulfo-KMUS (manufactured by Dojindo Laboratories), peroxidase (POD) (manufactured by Roche Diagnostics K.K.) was maleimided, and the unreacted Sulfo-KMUS was removed by a Sephadex G-25 column to obtain maleimide-modified POD. The prepared Fab' and maleimide-modified POD were mixed and separated by a Cephacil S-100HR column to prepare a POD-labeled anti-hCG antibody. Using this, a third reagent having the following composition was prepared.

"Third reagent": 6 nmol/L POD-labeled anti-hCG antibody, 50 mM MES (pH 6.5), 150 mM sodium chloride, 2.0% BSA

### (iV) "Fourth reagent"

1.82 g (50 mM) of 2-amino-2-hydroxymethyl-1,3-propanediol Tris(hydroxymethyl)aminomethane (manufactured by FUJIFILM Wako Pure Chemical Corporation), 0.690 mL (0.069%) of 5 N-HCl (manufactured by FUJIFILM Wako Pure Chemical Corporation), 0.877 g (150 mM) of sodium chloride (manufactured by FUJIFILM Wako Pure Chemical Corporation), 8.57 mg (1 mM) of sodium orthovanadate (manufactured by Sigma-Aldrich Japan G.K.), 0.1 g (5 mM) of sodium luminol (manufactured by FUJIFILM Wako Pure Chemical Corporation), and BiPCA (manufactured by Tokyo Chemical Industry Co., Ltd.) were dissolved in 100 mL of purified water such that the concentration of BiPCA was 0.2 mM, thereby preparing a fourth reagent.

### (V) "Fifth reagent"

"Fifth reagent": 68 µL/L of phosphoric acid (manufactured by FUJIFILM Wako Pure Chemical Corporation), 4 mM EDTA-2Na (manufactured by Dojindo Laboratories), 335 µL/L hydrogen peroxide (manufactured by FUJIFILM Wako Pure Chemical Corporation)

### 2. Preparation of blank sample

10.46 g (50 mM) of bis(2-hydroxyethyl)iminotris(hydroxymethyl)methane (manufactured by FUJIFILM Wako Pure Chemical Corporation), 1.7 mL (0.17%) of 5N HCl (manufactured by FUJIFILM Wako Pure Chemical Corporation), and 20 g (2.0%) of bovine serum albumin (manufactured by Sigma-Aldrich Japan G.K.) were dissolved in 1,000 mL of purified water to prepare a blank sample.

### 3. Preparation of measurement sample (hCG)

Human Chorionic Gonadotropin (manufactured by Meridian Japan Co., Ltd.) was dissolved in 10 mL of the blank sample prepared in "2. Preparation of blank sample" to prepare a high-unit hCG solution of 1,000 mIU/mL. Using the blank sample manufactured in "2. Preparation of blank sample" and the high-unit hCG solution, 0, 10, 20,000, and 30,000 mIU/mL hCG solutions were prepared as measurement samples.

### 4. Evaluation of luminescent reagent

Using the first to fifth reagents prepared in "1. Preparation of luminescent reagent (hCG)" and Accuraseed B/F separation solution (manufactured by FUJIFILM Wako Pure Chemical Corporation), the luminescence intensity of each solution was measured by an automatic chemiluminescence enzyme immunoassay analyzer Accuraseed (manufactured by FUJIFILM Wako Pure Chemical Corporation) according to the following procedure. 50 µL of the first reagent added to the reaction cuvette was heated at 67°C for 20 seconds while magnetic particles were collected using a neodymium magnet, and the supernatant was removed. Subsequently, 140 µL of the second reagent and 10 µL of the measurement sample (hCG solution) at a concentration of 0, 10, 20,000, or 30,000 mIU/mL were added thereto, and the mixture was stirred and heated at 37°C for 3 minutes. After completion of the heating, the magnetic particles were collected using a neodymium magnet, a reagent other than the magnetic particles was removed, and the magnetic particles were washed three times with a washing solution. Subsequently, 50 µL of a third reagent was added thereto, the mixture was heated at 37°C for 3 minutes. After completion of the heating, the magnetic particles were collected using a neodymium magnet, a reagent other than the magnetic particles was removed, and the magnetic particles were washed three times with a washing solution. After completion of the washing, 100 µL of a fourth reagent and 100 µL of a fifth reagent were added, reacted at 37°C for 20 seconds, and then the luminescence intensity was measured.

The low sensitivity and the high sensitivity were calculated from the obtained luminescence intensity. The low sensitivity was calculated from (luminescence intensity of 10 mIU/mL hCG solution)/(luminescence intensity of blank sample), and the high sensitivity was calculated from (luminescence intensity of 30,000 mIU/mL hCG solution)/(luminescence intensity of 20,000 mIU/mL hCG solution). The results are shown in Table 4.

### <Comparative Example 1> Evaluation of thiazole phenol (TP) in measurement of hCG

The luminescent reagent was prepared and evaluated by the same method as in Example 1, except as described below.

As the fourth reagent, thiazole phenol (TP) (FUJIFILM Wako Pure Chemical Corporation) was dissolved such that the concentration was 0.2 mM, instead of BiPCA (manufactured by Tokyo Chemical Industry Co., Ltd.) in (iV) of Example 1. The results are shown in Table 4.

### <Example 2> Evaluation of BiPCA in measurement of E2

### 1. Preparation of luminescent reagent (E2)

The luminescent reagent was prepared by the same method as in "1. Preparation of luminescent reagent (hCG)" of Example 1, except as described below.

In the "first reagent", instead of Mab to hCG beta (manufactured by Meridian Japan Co., Ltd.), which is an anti-hCG antibody, anti-E2 antibody-immobilized magnetic particles were prepared using Sheep Monoclonal Antibodies (SMAs) to E2 5A11 (manufactured by Bioventix plc) as an anti-E2 antibody. Instead of the anti-hCG antibody-immobilized magnetic particles in the "first reagent", the obtained anti-E2 antibody-immobilized magnetic particles were used.

In addition, in the "third reagent", a POD-labeled anti-immunocomplex M19-1G antibody was prepared by using an anti-immunocomplex M19-1G antibody produced by a hybridoma prepared by a method described later was used instead of MAB <HCG> M-INN22 IgG (manufactured by Roche Diagnostics K.K.), which is an anti-hCG antibody. In addition, instead of the POD-labeled anti-hCG antibody in the "third reagent", the obtained POD-labeled anti-immunocomplex M19-1G antibody was used.

The hybridoma was prepared by the following method. The complex of E2, which is an immunogen, and an anti-E2 antibody was injected into a mouse, which is an immunized animal, to immunize the mouse, and then lymphocytes were collected. The obtained lymphocytes and myeloma were fused with each other to screen for a hybridoma cell that produced a desired antibody. After cloning, a desired antibody-producing hybridoma was prepared.

### 2. Preparation of blank sample

The blank sample was prepared by the same method as in "2. Preparation of blank sample" of Example 1.

### 3. Preparation of measurement sample (E2)

β-Estradiol (manufactured by FUJIFILM Wako Pure Chemical Corporation) was dissolved in 10 mL of the blank sample prepared in "2. Preparation of blank sample" to prepare a high-unit E2 solution of 10,000 pg/mL. Using the blank sample prepared in "2. Preparation of blank sample" and the high-unit E2 solution, 0, 200, 2,000, and 3,000 pg/mL E2 solutions were prepared as measurement samples.

### 4. Evaluation of luminescent reagent

Using the luminescent reagent, the luminescence intensities were measured by the same method as in "4. Evaluation of Luminescent Reagent" of Example 1, and the low sensitivity and the high sensitivity were calculated. The low sensitivity was calculated from (luminescence intensity of 200 pg/mL E2 solution)/(blank luminescence intensity), and the high sensitivity was calculated from (luminescence intensity of 3,000 pg/mL E2 solution)/(luminescence intensity of 2,000 pg/mL E2 solution). The results are shown in Table 4 and FIG. 1.

### (Examples 3 to 17) Evaluation of various compounds in measurement of E2>

The luminescent reagent was prepared and evaluated by the same method as in Example 2, except as described below. Instead of BiPCA (manufactured by Tokyo Chemical Industry Co., Ltd.) in the fourth reagent of Example 2, 4-(pyridin-2-yl)phenol (manufactured by Combi-Blocks), 4-(oxazol-4-yl)phenol (manufactured by Combi-Blocks), 4-(1H-pyrrol-1-yl)phenol (manufactured by Enamine), and an enhancer manufactured in the synthesis example were used and dissolved such that the concentration was 0.2 mM (the reaction concentration at the time of measurement was 0.1 mM), and the mixture was used as the fourth reagent. The structures of the enhancers used in Examples 3 to 17 are shown below. The results are shown in Table 4.

**[Table 2]**

| Example | Structure | Example | Structure |
|---|---|---|---|
| Example 3 | 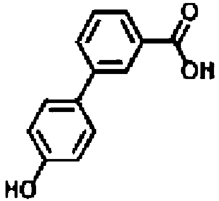 | Example 7 | 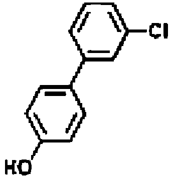 |
| Example 4 | 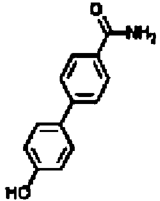 | Example 8 | 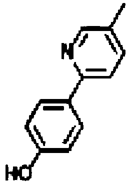 |
| Example 5 | 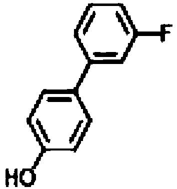 | Example 9 | 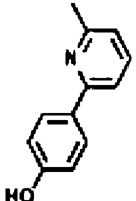 |
| Example 6 | 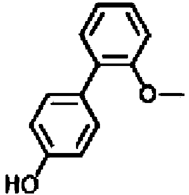 | Example 10 | 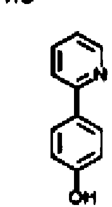 |
| Example 11 | 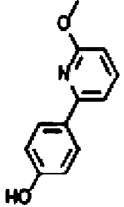 | Example 15 | 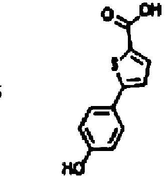 |
| Example 12 | 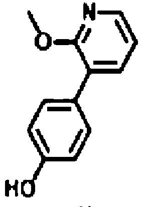 | Example 16 | 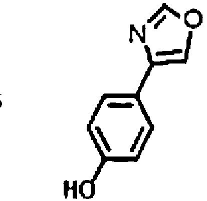 |
| Example 13 | 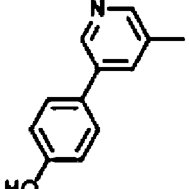 | Example 17 | 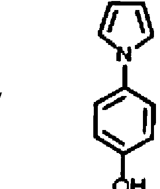 |
| Example 14 | 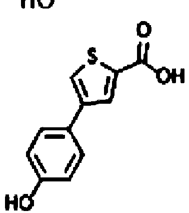 | | |

### <Comparative Example 2> Evaluation of thiazole phenol (TP) in measurement of E2

The luminescent reagent was prepared and evaluated by the same method as in Example 2, except as described below.

As the fourth reagent, thiazole phenol (TP) (FUJIFILM Wako Pure Chemical Corporation) was dissolved such that the concentration was 0.2 mM, instead of BiPCA (manufactured by Tokyo Chemical Industry Co., Ltd.) in Example 2. The results are shown in Table 4 and FIG. 1.

### (Comparative Examples 3 and 4) Evaluation of various compounds in measurement of E2

The luminescent reagent was prepared and evaluated by the same method as in Example 2, except as described below. Instead of BiPCA (manufactured by Tokyo Chemical Industry Co., Ltd.) in the fourth reagent of Example 2, the compounds shown in Table 3 (all of which are manufactured by Tokyo Chemical Industry Co., Ltd.) were dissolved such that the concentration was 0.2 mM (the reaction concentration at the time of measurement was 0.1 mM), and the mixture was used as the fourth reagent. The results are shown in Table 4.

**[Table 3]**

| Example | Name | Structure |
|---|---|---|
| Comparative Example 3 | PBP | 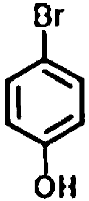 |
| Comparative Example 4 | PIP | 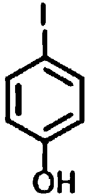 |

### (Comparative Examples 5 and 6) Evaluation of BiPCA in measurement of HBs antigen

### 1. Adjustment of luminescent reagent (HBs antigen)

The luminescent reagent was prepared by the same method as in "1. Preparation of luminescent reagent (hCG)" of Example 1, except as described below.

In the "first reagent", anti-HBs antigen antibody-immobilized magnetic particles was prepared by using the anti-HBs antibody produced by a hybridoma prepared by a method described later was used instead of Mab to hCG beta (manufactured by Meridian Japan Co., Ltd.), which is an anti-hCG antibody. Instead of the anti-hCG antibody-immobilized magnetic particles in the "first reagent", the obtained anti-HBs antigen antibody-immobilized magnetic particles were used.

The hybridoma was prepared by the following method. The HBs antigen, which is an immunogen, was injected into a mouse, which is an immunized animal, to immunize the mouse, and then lymphocytes were collected. The obtained lymphocytes and myeloma were fused with each other to screen for a hybridoma cell that produced a desired antibody. After cloning, a desired antibody-producing hybridoma was prepared.

In the "third reagent", a POD-labeled anti-hCG antibody was prepared by using an anti-hCG antibody produced by a hybridoma prepared by the above-described method was used instead of MAB <HCG> M-INN22 IgG (manufactured by Roche Diagnostics K.K.), which is an anti-hCG antibody. Instead of the POD-labeled anti-hCG antibody in the "third reagent", the POD-labeled anti-HBs antibody was used.

In the "fourth reagent", L-012 (manufactured by FUJIFILM Wako Pure Chemical Corporation) was used instead of sodium luminol (manufactured by FUJIFILM Wako Pure Chemical Corporation).

### 2. Preparation of blank sample

The blank sample was prepared by the same method as in "2. Preparation of blank sample" of Example 1.

### 3. Preparation of sample (HBs antigen)

HBs antigen (ad) (manufactured by Trina Bioreactives AG) was dissolved in 10 mL of the blank sample prepared in "2. Preparation of blank sample" to prepare a high-unit HBs antigen solution of 2,000 IU/mL. Using the blank sample prepared in "2. Preparation of blank sample" and the high-unit HBs antigen solution, 0, 0.2, 50, and 300 IU/mL HBs antigen solutions were prepared as measurement samples.

### 4. Evaluation of luminescent reagent

Using the luminescent reagent, the luminescence intensity in a case of measuring each luminescent reagent using an automatic chemiluminescence enzyme immunoassay analyzer Accuraseed (manufactured by FUJIFILM Wako Pure Chemical Corporation) was measured, and the low sensitivity and the high sensitivity were calculated. The low sensitivity was calculated from (luminescence intensity of 0.2 IU/mL HBs antigen)/(blank luminescence intensity), and the high sensitivity was calculated from (luminescence intensity of 300 IU/mL HBs antigen)/(luminescence intensity of 50 IU/mL HBs antigen). The results are shown in Table 4.

### <Examples 18 and 19> Evaluation of BiPCA in measurement of E2

The luminescent reagent was prepared by the same method as in Example 2, except that BiPCA (manufactured by Tokyo Chemical Industry Co., Ltd.) was used at the following concentration instead of 0.2 mM BiPCA in the "fourth reagent", and the evaluation was performed. The results are shown in Table 4.

Example 18: 0.3 mM BiPCA in "fourth reagent" (reaction concentration at the time of measurement is 0.15 mM)

Example 19: 0.6 mM BiPCA in "fourth reagent" (reaction concentration at the time of measurement is 0.30 mM)

### <Examples 20 and 21> Evaluation of BiPCA in measurement of hCG

The luminescent reagent was prepared by the same method as in Example 1, except that BiPCA (manufactured by Tokyo Chemical Industry Co., Ltd.) was used at the following concentration instead of 0.2 mM BiPCA in the "fourth reagent", and the evaluation was performed. The results are shown in Table 4.

Example 20: 0.3 mM BiPCA in "fourth reagent" (reaction concentration at the time of measurement is 0.15 mM)

Example 21: 0.6 mM BiPCA in "fourth reagent" (reaction concentration at the time of measurement is 0.30 mM)

**[Table 4]**

| | Measurement target | Enhancer | Concentration of enhancer | Low sensitivity | High sensitivity |
|---|---|---|---|---|---|
| Comparative Example 1 | hCG | TP | 0.10 | 19.78 | 0.99 |
| Example 1 | hCG | BiPCA | 0.10 | 36.97 | 1.23 |
| Comparative Example 2 | E2 | TP | 0.10 | 30.67 | 0.87 |
| Comparative Example 3 | E2 | PBP | 0.10 | 8.15 | 1.62 |
| Comparative Example 4 | E2 | PIP | 0.10 | 17.91 | 1.68 |
| Example 2 | E2 | BiPCA | 0.10 | 41.87 | 1.61 |
| Example 3 | E2 | Compound shown in Table 2 | 0.10 | 37.48 | 1.63 |
| Example 4 | E2 | Compound shown in Table 2 | 0.10 | 32.47 | 1.64 |
| Example 5 | E2 | Compound shown in Table 2 | 0.10 | 36.56 | 1.31 |
| Example 6 | E2 | Compound shown in Table 2 | 0.10 | 38.72 | 1.30 |
| Example 7 | E2 | Compound shown in Table 2 | 0.10 | 25.97 | 1.48 |
| Example 8 | E2 | Compound shown in Table 2 | 0.10 | 56.23 | 1.49 |
| Example 9 | E2 | Compound shown in Table 2 | 0.10 | 44.76 | 1.69 |
| Example 10 | E2 | Compound shown in Table 2 | 0.10 | 40.33 | 1.86 |
| Example 11 | E2 | Compound shown in Table 2 | 0.10 | 34.51 | 1.81 |
| Example 12 | E2 | Compound shown in Table 2 | 0.10 | 40.38 | 1.41 |
| Example 13 | E2 | Compound shown in Table 2 | 0.10 | 39.94 | 1.71 |
| Example 14 | E2 | Compound shown in Table 2 | 0.10 | 42.67 | 1.60 |
| Example 15 | E2 | Compound shown in Table 2 | 0.10 | 38.30 | 1.76 |
| Example 16 | E2 | Compound shown in Table 2 | 0.10 | 25.22 | 1.60 |
| Example 17 | E2 | Compound shown in Table 2 | 0.10 | 29.07 | 1.79 |
| Comparative Example 5 | HBs antigen | TP | 0.10 | 6.69 | 5.94 |
| Comparative Example 6 | HBs antigen | BiPCA | 0.10 | 1.87 | 7.04 |
| Example 18 | E2 | BiPCA | 0.15 | 42.21 | 1.62 |
| Example 19 | E2 | BiPCA | 0.30 | 49.20 | 1.59 |
| Example 20 | hCG | BiPCA | 0.15 | 37.75 | 1.25 |
| Example 21 | hCG | BiPCA | 0.30 | 38.66 | 1.22 |

It is desirable that the concentration of the measurement target substance and the luminescence intensity, at least in a concentration range of the measurement target substance required for each measurement target substance, exhibit an increase in luminescence intensity with an increase in the concentration of the measurement target substance. However, from Table 4 and FIG. 1, it was found that, in a case where hCG (Comparative Example 1) or E2 (Comparative Example 2) was measured using TP, which was known as an enhancer for enhancing chemiluminescence, the luminescence intensity did not reach the luminescence intensity expected in a case where hCG or E2 was contained at a high concentration in a concentration range of hCG or E2 required for measurement, and the luminescence intensity did not increase, whereby the amount of the measurement target substance could not be accurately measured.

Therefore, with a case of using TP taken as a reference, compounds were investigated that significantly improves the high sensitivity compared with TP while providing low sensitivity equivalent to that obtained when TP is used. As a result, in the compounds used in Comparative Examples 3 and 4, the effect of enhancing chemiluminescence as an enhancer was present, and the high sensitivity was improved as compared with TP of Comparative Example 2, but the low sensitivity was significantly reduced. On the other hand, in the compounds used in Examples 1 to 17, the low sensitivity was the same as that of TP of Comparative Example 2, and the high sensitivity was significantly improved to 1.2 times or more. As a result of Examples 18 to 21, the same effect was observed even in a case where the reaction concentration of BiPCA at the time of measurement was changed from 0.1 mM to 0.15 mM or 0.3 mM.

In addition, as a result of Comparative Examples 5 and 6, in a case where BiPCA was used in the measurement of the HBs antigen, the high sensitivity was slightly improved as compared with TP, but the low sensitivity was significantly reduced. That is, the effect of improving the high sensitivity while maintaining the low sensitivity (the same as in a case of using TP) by the compound according to the present invention was confirmed only in the measurement of hCG and E2.

## Claims

1. A method for measuring an antigen contained in a test sample, the antigen being human chorionic gonadotropin or estradiol, the method comprising:
reacting, in a presence of one or more compounds represented by Formula (1),
(1) an antigen having peroxidase bound to the antigen through a binding substance that recognizes the antigen, or
an antigen having peroxidase bound to the antigen through a binding substance that recognizes a complex of the antigen and a binding substance that recognizes the antigen,
(2) a chemiluminescent substance, and
(3) an oxidizing agent, 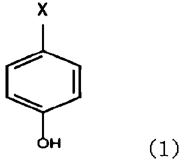
in the formula, X is
a phenyl group that is unsubstituted or has a substituent,
a pyridyl group that is unsubstituted or has a substituent,
an oxazolyl group that is unsubstituted or has a substituent,
a thiophenyl group that is unsubstituted or has a substituent, or
a pyrrolyl group that is unsubstituted or has a substituent, and
the substituent is a carboxyl group, an amide group, a halogen atom, an alkoxy group having 1 to 5 carbon atoms, or an alkyl group having 1 to 5 carbon atoms.

2. The measurement method according to claim 1,
wherein X is
a phenyl group having a carboxyl group, an amide group, a halogen atom, or an alkoxy group having 1 to 5 carbon atoms,
a pyridyl group that is unsubstituted or has an alkyl group having 1 to 5 carbon atoms or an alkoxy group having 1 to 5 carbon atoms,
an oxazolyl group that is unsubstituted or has an alkyl group having 1 to 5 carbon atoms or an alkoxy group having 1 to 5 carbon atoms,
a thiophenyl group that is unsubstituted or has a carboxyl group, or
a pyrrolyl group that is unsubstituted or has an alkyl group having 1 to 5 carbon atoms or an alkoxy group having 1 to 5 carbon atoms.

3. The measurement method according to claim 1,
wherein X is a phenyl group having a carboxyl group, an amide group, a halogen atom, or an alkoxy group having 1 to 5 carbon atoms.

4. The measurement method according to claim 1,
wherein the antigen having peroxidase bound to the antigen through a binding substance that recognizes the antigen is an antigen obtained by reacting the antigen with a first binding substance that recognizes the antigen to form a complex, and subsequently reacting the complex with a second binding substance that recognizes the antigen, the second binding substance having peroxidase bound to the antigen.

5. The measurement method according to claim 1,
wherein the antigen having peroxidase bound to the antigen through a binding substance that recognizes a complex of the antigen and a binding substance that recognizes the antigen is an antigen obtained by reacting the antigen with a first binding substance that recognizes the antigen to form a complex, and subsequently reacting the complex with "a third binding substance that recognizes the complex of the antigen and the first binding substance that recognizes the antigen", the third binding substance having peroxidase bound to the antigen.

6. The measurement method according to any one of claims 1 to 5,
wherein a concentration of the compound represented by Formula (1) is 10 µmol/L or more and 500 µmol/L or less.

7. The measurement method according to any one of claims 1 to 5,
wherein the chemiluminescent substance is luminol, a luminol derivative, 8-amino-5-chloro-7-phenylpyrido[3,4-d]pyridazine-1,4(2H,3H)-dione sodium salt, or salts thereof.

8. The measurement method according to claim 7,
wherein a concentration of the chemiluminescent substance is 0.1 mmol/L or more and 10 mmol/L or less.

9. The measurement method according to any one of claims 1 to 5,
wherein the oxidizing agent is hydrogen peroxide.

10. A kit for measuring human chorionic gonadotropin or estradiol, the kit comprising:
peroxidase to which a binding substance that recognizes an antigen is bound;
a chemiluminescent substance;
an oxidizing agent; and
one or more compounds represented by Formula (1), 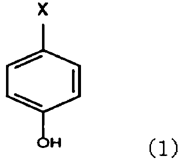
in the formula, X is
a phenyl group that is unsubstituted or has a substituent,
a pyridyl group that is unsubstituted or has a substituent,
an oxazolyl group that is unsubstituted or has a substituent,
a thiophenyl group that is unsubstituted or has a substituent, or
a pyrrolyl group that is unsubstituted or has a substituent, and
the substituent is a carboxyl group, an amide group, a halogen atom, an alkoxy group having 1 to 5 carbon atoms, or an alkyl group having 1 to 5 carbon atoms.

11. The kit according to claim 10, further comprising:
another binding substance that recognizes a measurement antigen, the other binding substance being different from the binding substance bound to the peroxidase; or
a solid-phase carrier on which the other binding substance is immobilized.

12. A kit for measuring human chorionic gonadotropin or estradiol, the kit comprising:
peroxidase to which a binding substance that recognizes a complex of an antigen and a binding substance that recognizes the antigen is bound;
a chemiluminescent substance;
an oxidizing agent; and
one or more compounds represented by Formula (1), 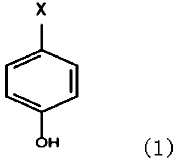
in the formula, X is
a phenyl group that is unsubstituted or has a substituent,
a pyridyl group that is unsubstituted or has a substituent,
an oxazolyl group that is unsubstituted or has a substituent,
a thiophenyl group that is unsubstituted or has a substituent, or
a pyrrolyl group that is unsubstituted or has a substituent, and
the substituent is a carboxyl group, an amide group, a halogen atom, an alkoxy group having 1 to 5 carbon atoms, or an alkyl group having 1 to 5 carbon atoms.

13. The kit according to claim 12, further comprising:
another binding substance that recognizes a measurement antigen, the other binding substance being different from the binding substance bound to the peroxidase; or
a solid-phase carrier on which the other binding substance is immobilized.
